# EUROPEAN PATENT APPLICATION

(11) **EP 4 575 544 A1**
(43) Date of publication of application: **25.06.2025**
(21) Application number: 23219863.0
(22) Date of filing: 22.12.2023
(51) Int. Cl.: G01R 33/567

(54) **A METHOD FOR ACQUIRING IMAGE DATA USING PILOT TONE**

(71) Applicant: Siemens Healthineers AG, 91301 Forchheim (DE)
(72) Inventor: Speier, Peter, 91056 Erlangen (DE); Bacher, Mario, 91227 Diepersdorf (DE); Huang, Yan Tu, Shenzhen, 518000 (CN)
(74) Representative: Siemens Healthineers Patent Attorneys

(57) **Abstract**

The invention is directed to a method for acquiring image data from a part of the human body subjected to a cardiac movement. The method comprising the steps of (a) transmitting a radiofrequency Tx Pilot Tone signal; (b) Receiving a Pilot Tone signal (108) comprising several channel signals (102); (c) Carrying out a blind source separation algorithm (122) on a training portion of the Pilot Tone signal and thereby determining weighting vectors (V)); (d) Selecting and storing at least two non-parallel weighting vectors (V) which allow to extract signal components (123), which represent cardiac movement, from the several channel signals; (e) Applying the weighting vectors to the further portions of the Pilot Tone signal (102) to obtain a multi-dimensional Pilot Tone signal (126) representing the cardiac movement, and (f) Using the multi-dimensional Pilot Tone signal (126) for triggering the acquisition of the image data.

## Description

The present invention relates to a method for acquiring image data in a radiological examination of a part of the human or animal body using a Pilot Tone method, to a computer program and a control unit for controlling a radiological imaging system, in particular a magnetic resonance imaging system.

Independent of the grammatical term usage, individuals with male, female or other gender identities are included within the term.

Patient movement or motion during a diagnostic examination or scan of medical data, in particular during radiological imaging, often causes artefacts in the acquired images. Especially Magnetic Resonance (MR) imaging is relatively slow, so that cyclical motions like respiration and cardiac movement, but also patient motion with respect to the measurement system, often occur during the scan. If the movement is known, the data acquisition can be triggered to a particular phase in the cyclical movement, or the acquired data can be corrected.

It is therefore known to acquire an ECG (electrocardiogram) of the patient during radiological imaging in order to trigger the data acquisition to a particular phase in the cardiac cycle. However, taking an ECG during a magnetic resonance imaging (MRI) scan presents difficulties, in particular because of the high magnetic fields, which may cause interferences in the ECG leads. Other methods for motion detection include breathing belts, the use of two-dimensional (2D) or three-dimensional (3D) cameras, radar, and RF (radiofrequency) based methods.

The basic principle of RF signal based methods optimized for use in MRI, also termed "Pilot Tone" methods, is to irradiate the body with an RF signal just outside the receive bandwidth of the MR data being acquired, but within the frequency range of the MR receive system, in particular within the oversampling bandwidth which is acquired during every readout. To transmit the RF signal, an RF transmitter may be installed in the radiological imaging modality, for example above the patient. The transmitted Pilot Tone signal interacts with the human body and is received via multiple antennas, for example the local RF coil(s). The received multi-channel Pilot Tone signal can be analysed in order to extract specific motion components. For example, Principal Component Analysis (PCA) techniques may be used, as disclosed in EP3413076A1, in order to separate orthogonal signal components from the multi-channel Pilot Tone signal. Generally, one signal component is extracted for each movement type, for example for the cardiac movement.

The advantage of RF based methods for detecting patient motion during magnetic resonance imaging (MRI) scans is that the RF transmission and reception infrastructure is already present.

In principle, the Pilot Tone method can be used for respiratory and cardiac triggering and gating. The cardiac application is especially challenging because the modulation of the Pilot Tone signal caused by the cardiac motion is rather small, in particular compared to the respiratory signal. In case the Pilot Tone method is used during an MR acquisition, the Pilot Tone signal may be affected by RF pulses, thereby compromising trigger stability.

Another limitation of cardiac Pilot Tone methods is that the cardiac signal does not show a clear R-wave, which could be used as a trigger time point. Generally, choices of trigger time points are limited to periods where the signal is changing significantly. If it remains constant or the change is so small that it cannot be reliably detected over a period of time, no trigger can be placed in this period. Therefore, current Pilot Tone implementations trigger well after the start of the cardiac contraction, using a trigger time point in the cardiac cycle approx. 200ms after the R-wave in the ECG. This can reduce stability, in particular in MR acquisitions which are preceded by a "dark blood" double inversion magnetisation preparation, which needs to be played out before the contraction, so that one needs to trigger the preparation before the start of contraction of the heart muscle during systole.

It is therefore an object of the present invention to improve the above-identified limitations of Pilot Tone methods on the trigger stability and on the possible trigger time points.

This object is met or exceeded by a method for acquiring image data in a radiological examination of a part of the human or animal body according to claim 1, a computer program according to claim 14 and a control unit for controlling a radiological imaging modality according to claim 15.

According to a first aspect, the invention is directed to a method for acquiring image data in a radiological examination of a part of the human or animal body, wherein the part is subjected to a cardiac movement. The method comprises the steps:
a) transmitting a radiofrequency Tx Pilot Tone signal,
b) Receiving a Pilot Tone signal from the body part via a radiofrequency receiver coil arrangement comprising several channels, wherein the received Pilot Tone signal comprises several channel signals associated with the several channels;
c) Carrying out a blind source separation algorithm on a training portion of the Pilot Tone signal and thereby determining weighting vectors to extract cardiac movement signals in the presence of other signals, wherein the weighting vectors allow to form weighted combinations of the several channel signals;
d) Selecting and storing at least two non-parallel weighting vectors which allow to extract signal components, which represent cardiac movement, from the several channel signals;
e) Applying the weighting vectors to the further portions of the Pilot Tone signal to obtain a multi-dimensional Pilot Tone signal representing the cardiac movement, wherein the multi-dimensional Pilot Tone signal has at least two dimensions;
f) Using the multi-dimensional Pilot Tone signal for controlling, in particular triggering, the acquisition of the image data, or for retrospectively gating or correcting the acquired image data.

The invention has realised that the cardiac signature in the Pilot Tone signal is not limited to a single component. Instead, at least two signal components contribute to the cardiac modulation. However, this is not reflected in prior art methods using a Pilot Tone method to trigger the acquisition of image data. Rather, so far only the strongest signal component representing cardiac movement was used, whereas the other component or components were ignored. The invention therefore proposes to improve the quality of the Pilot Tone signal used for controlling the acquisition, as well as the trigger stability, by extending the dimensionality of the cardiac Pilot Tone signal from a one-dimensional signal to a multi-dimensional signal and utilizing these additional dimensions in the trigger detection. The number of dimensions is herein referred to as N.

The method of the invention is carried out during the acquisition of image data in a radiological examination of a part of the human or animal body, wherein the part is subjected to cardiac movement. The multi-dimensional Pilot Tone signal obtained by the inventive method is preferably used to control the image data acquisition, in particular for triggering the acquisition to particular phases in the cardiac cycle, or for. Alternatively, the multi-dimensional Pilot Tone signal can be used to retrospectively gate or correct the acquired image data. In particular, if the acquired image data can be assigned to particular cardiac phases, the image can be reconstructed using retrospective motion correction techniques.

The radiological examination can be any imaging examination using a medical imaging modality, for example computed tomography (CT), x-ray imaging, ultrasound imaging, or positron emission spectroscopy. Preferably, the radiological examination is a magnetic resonance imaging examination. This is advantageous because the RF infrastructure required for transmitting and receiving the Pilot Tone signal is already present. The Pilot Tone signal is preferably transmitted and acquired during the entire imaging procedure, in particular during an MR scan, or at least during relevant parts thereof. The part of the human or animal body, of which image is to be acquired, is subjected to cardiac movement. The part may be for example the heart, the lungs, the brain, peripheral blood vessels, an organ within the abdomen, thorax, or any organ affected by a pulsating blood.

The method comprises transmitting an RF transmit (Tx) Pilot Tone signal, preferably via at least one RF transmit antenna, also referred to as PT generator. Advantageously, the RF transmit antenna includes a magnetic near field generating loop. The Tx Pilot Tone (PT) signal may be a continuous wave (CW) signal and may be generated by an independent continuous wave RF source. It may also be a pulsed signal. Preferably, the frequency of the Tx PT signal is still within the bandwidth of the MR image acquisition, if the method is applied during an MR examination, to avoid interference with the MRI data. The frequency of the transmitted Pilot Tone signal may be between about 20 and 2000 kHz, preferably between 200 and 1000 kHz, more preferred between 500 and 1000 kHz away from the centre frequency of the MR signal, in particular the MR signal acquired in the MRI examination. The Pilot Tone signal interacts with the body part and the received PT signal is therefore modulated by its movement.

The RF receiver coil arrangement comprises several channels, as is common in MR receiver coil arrangements, e.g. local coils and coil arrays. Each element acquires a separate so-called channel signal. Thus, the received Pilot Tone signal comprises several channel signals associated with the several channels of the RF receiver coil arrangement. Preferably, the invention uses 4 to 8, preferably 8 to 64 channels. Different types of movement usually affect the channels to different degrees, depending on the orientation and position of the moving body part with respect to the coil element and to the PT generator. It is therefore necessary to combine the several channel signals in an intelligent way in order to extract particular movement types such as cardiac movement and/or respiratory movement.

The method of this invention is particularly directed to extract the cardiac movement, which means any movement caused by the cyclical contraction of the heart muscles, be it in the heart itself or in other body parts affected by it, in particular by arterial blood vessels.

In order to extract the signal components pertaining to the cardiac movement, a blind source separation (BSS) algorithm is carried out on a training portion of the Pilot Tone signal. The extracted signal components are preferably independent, wherein "independent" is to be understood broadly as a general term to describe components found by any BSS method, including Principal Component Analysis (PCA). It must not be understood as limited to components found by Independent Component Analysis (ICA). The principle of blind source separation (BSS) is the separation of a set of source signals from a set of mixed input signals, without or with minimal aid of information about the source signals or the mixing process. BSS aims at finding an independent set of vectors (herein also termed "weighting vectors"), onto which the input signals can be transformed. The data that is projected or mapped onto each vector corresponds to an independent source. Since the method involves projecting the data onto a set of axes/vectors which are determined by the nature of the data, the method is termed blind source separation ("blind" because the projection vectors are determined without of the use of any prior knowledge of the data structure). The BSS algorithm is applied on a training portion of the Pilot Tone signal. The training portion is preferably a short signal portion covering a few (e.g. 1 to 20) heartbeats acquired before or at the beginning of the radiological examination. Alternatively, e.g. when the Pilot Tone signal is to be applied retrospectively, the training portion may cover the complete or a part of the Pilot Tone signal acquired during the examination.

The BSS algorithm is thus used to determine weighting vectors in order to extract cardiac movement signals in the presence of other signals, wherein the weighting vectors allow to form weighted combinations of the several channel signals. The weighting vectors are nonparallel, which means that they allow to extract different signal components, in particular independent signal components, wherein the measure of independence may be defined in various ways, according to different embodiments. In an embodiment, the nonparallel weighting vectors allow to extract statistically independent signal components. Example techniques use for determining the weighting vectors will be explained below in more detail.

According to the invention, at least two nonparallel weighting vectors are selected and stored, which allow to extract at least N=2 signal components which represent cardiac movement, from the several channel signals. Thus, according to the invention, not one, but at least two - preferably independent - signal components are used to describe the cardiac movement. Once the weighting vectors have been determined from the training portion, the weighting vectors are applied to the further portions of the Pilot Tone signal to obtain the multi-dimensional Pilot Tone signal representing the cardiac movement. This signal is used for controlling, in particular triggering, the acquisition of the image data, or for retrospectively gating or correcting the acquired image data. Thereby, higher dimensional components are added to the Pilot Tone signal and can be used for triggering the image acquisition. This has the advantage that the usage of the input signal is maximised, compared to the prior art approach, where the energy in the higher components is not used. This will allow to reduce the amplitude of the transmitted Pilot Tone signal without loss of stability, and reduce the likelihood of signal to noise ratio loss due to activation of the compander architecture in the receiver. Alternatively, trigger stability with respect to noise and RF artefacts can be increased for a given PT amplitude.

In addition, it has been found that the higher dimensional signal components show activity at different time points compared to the first (strongest) component. This will enable to extract new trigger time points in the cardiac interval from the PT signal. Especially, trigger time points earlier in the cardiac cycle, i.e. closer and even before the R-wave, will be possible. This reduces the prior art problems specifically when using MR sequences requiring magnetisation preparation, such as for dark blood imaging. The invention thereby allows to improve on a promise of the Pilot Tone method, namely that it should enable multiple trigger points in the cardiac cycle, for example at end systole or early diastole. The Pilot Tone signal has been found to follow the cardiac volume curve closely without any lag, except processing delays. However, with only a single signal component, the only practical prospective trigger time points for typical cardiac Pilot Tone signals was during mid and end systole or early diastole. By including further higher order signal components into the signal analysis, it is possible to extract many more useful trigger time points within the cardiac cycle, especially ones close or even preceding the R-wave.

According to an embodiment, the multi-dimensional Pilot Tone signal representing the cardiac movement has between 2 and 5, preferably 2 or 3, dimensions, i.e. N=2-5, preferably 2-3.

With current PT signal strengths, it is believed that up to N=5 signal components may maximally be used for cardiac triggering. As the higher dimensional signal components will likely have lower energy, it is unlikely that any higher order components will be valuable. Preferably, the strongest 2 or 3 signal components will be extracted using the weighting vectors, thus the multi-dimensional signal will be 2- or 3-dimensional.

According to an embodiment, the blind source separation algorithm may use techniques such as Principal Component Analysis (PCA), using e.g. singular value decomposition, or Independent Component Analysis (ICA). In both PCA and ICA, one attempts to find a set of axes or weighting vectors, which are independent of one another in some sense. One assumes that there are a set of independent sources in the data, but does not assume their exact properties. By defining some measure of independence, it is then possible to decorrelated the data by maximising this measure between projections onto each axis of the new space, into which the data is transformed. Thus, the independent signal components (which represent independent movement types) are obtained by applying the weighting vectors onto the received Pilot Tone channel signals.

According to an embodiment, the blind source separation algorithm utilises one or more Principal Component Analysis (PCA) operations. In PCA, the optimized variable is the variance. By maximizing variance, PCA operations lead to a set of orthogonal signal components. PCA operations are useful in particular to reduce the complexity of the BSS problem. For example, a dimensionality reduction can be performed by applying a PCA based on either eigenvalue or singular value decomposition, and using only the largest principle component's singular values.

For example, a PCA may be used to remove the respiratory signal component from the Pilot Tone signal. This may for example be performed as follows.

The received Pilot Tone signal is optionally pre-processed by low-pass or bandpass filtering, in order to avoid aliasing of high-frequency noise. This may optionally be followed by down-sampling, in particular during portions of the PT signal acquired outside MT measurements. During MR measurements, a high sample rate is preferably maintained because the signal is frequently corrupted by the RF pulses of the MR measurement. The pre-processing is optional because it increases the signal to noise ratio but at the cost of additional time delay. Further, the pre-processed signal may be subjected to a normalisation step, in which the phases of all channels are normalised to a reference phase. The phase normalisation may be achieved by multiplying with the complex conjugate of the reference channel, i.e. one of the channels is selected as the reference channel.

According to this embodiment, the (optionally normalised) Pilot Tone signal is further processed to reduce all other signals but the respiratory one. This may be achieved by first bandpass filtering the signal to the respiratory frequency range, and then applying a PCA operation, in which the strongest principle components are extracted. The first few strongest components represent respiratory motion, and this part of the input signal (i.e. the received Pilot Tone signal) is then removed. On the remaining signal, an appropriate BSS algorithm, e.g. PCA or ICA, is repeated for the cardiac movement, preferably on data filtered to the cardiac frequency range. Thereby, the weighting vectors which extract the cardiac movement signals may be determined. According to the invention, at least 2 signal components representing cardiac movement are extracted. For example, the real part and the imaginary part of the first component, i.e. the component corresponding to the largest eigenvalue, may be the extracted as the first and second signal component.

According to an embodiment, the blind source separation algorithm utilises one or more Independent Component Analysis (ICA) operations. ICA is based on the assumption that the individual signal components are statistically independent from each other. Thus, ICA operations find the independent components by maximising a measure of statistical independence of estimated components. The measure of independence between the axes may for example be based on non-Gaussianity, and the axes are not necessarily orthogonal. Thus, solving the ICA problem can be formulated as a problem of e.g. minimising the mutual information or maximising the non-Gaussianity. Gradient descent methods or other optimization techniques may be used.

According to an embodiment, the blind source separation algorithm calculates a demixing matrix, where the demixing matrix extracts independent, preferably statistically independent, components from the several channel signals. Whatever BSS analysis method is used, e.g.,. PCA or ICA, it is useful to calculate a demixing matrix. The demixing matrix may be applied to the further portions of the Pilot Tone signal in order to extract the independent signal components from the several channel signals of the received and optionally pre-processed Pilot Tone signal. Depending on the implementation, the demixing matrix can be either complex or real valued. In practice it is sufficient to store and apply to the subsequent data only the parts of the demixing matrix that are necessary to calculate the desired components.

According to an embodiment, the blind source separation algorithm is used to detect the strongest independent component corresponding to the cardiac movement, and the method comprises using the strongest independent component to retrospectively analyse the training portion of the Pilot Tone signal, in particular to average the Pilot Tone signal over a plurality of cardiac intervals, the cardiac intervals having been determined from the strongest statistically independent component, and detecting at least one further independent component corresponding to the cardiac movement from the retrospective analysis.

This embodiment is highly useful, because usually the strongest independent component corresponding to the cardiac movement may be reliably detected. The higher order components (i.e. the 2^{nd}, 3^{rd},... strongest signal components) are harder to detect, as their energy is lower. However, it is known that they have exactly the same (varying) periodicity as the 1^{st}, strongest component. Therefore, one can stabilise their detection after retrospective trigger detection on the 1^{st} component. This can in particular be done by averaging the received Pilot Tone signal over a plurality of cardiac intervals. In particular, the averaging is done over the exact cardiac intervals, the length of which vary over time, and which have been determined from the strongest independent component. After the averaging, the signal can be subjected to another PCA or ICA operation in order to detect at least one further independent component corresponding to the cardiac movement from the averaged data. Other forms of retrospective analysis may consist in removing the strongest independent signal component from the data.

According to an embodiment, the channel signals of the received Pilot Tone signal are complex-valued, and wherein the weighting vectors each extract a real or an imaginary part of an independent signal component.

Since the RF coil in generally also detect the phase, the several channel signals may be complex valued. It has been discovered that it is useful to treat real and imaginary parts as separate signal components, since they may in fact contain independent information from one another. Therefore, preferably the weighting vectors each extract either a real or an imaginary part of an independent signal component, making this real or imaginary part one separate signal component which may be used in the multi-dimensional Pilot Tone signal. In other words, a signal component having a real and imaginary part is a two-dimensional Pilot Tone signal for the purposes of this invention. Evidently, a real and imaginary part must not necessarily belong to the same independent signal component, as extracted for example by ICA or PCA. It is also possible that the multi-dimensional Pilot Tone signal comprises 2 real parts or 2 imaginary parts of different independent signal components.

According to an embodiment, the channel signals of the received Pilot Tone signal are complex-valued, and wherein the channel signals are rotated in the complex plane before carrying out the blind source separation algorithm, in particular rotated so that a mean of the rotated signal lies on one of the diagonals of the complex plane. This embodiment is advantageous because the real and imaginary components thereby become equally strong, making the channel signals numerically easier to process, for example by PCA or ICA. In particular, the channel signals may be rotated in the complex plane so that a mean of the rotated signal is on one of the diagonals of the complex plane. In other words, abs(real(PT)) = abs (imag(PT)) .

According to an embodiment, the channel signals of the received Pilot Tone signal are complex and the blind source separation algorithm is real-valued, and wherein the method comprises generating a real-valued matrix in which the real and imaginary parts of the complex channel signals form separate channels, and performing a blind source separation algorithm on the real-valued matrix.

As stated above, that the two or three strongest cardiac components are captured in the complex-valued strongest signal component is a special case. In general, the 2 or 3 cardiac components of relevance are distributed over two principal components. Therefore, one needs a way to separate and capture the 1st 2 to 5, preferably 2 to 3, cardiac signal components in all cases. This can be done in several ways. In one embodiment, the complex valued DSS is substituted by a real valued BSS (for example composite real valued PCA), where the real and imaginary parts of the complex channel signals are used to generate a real valued matrix without information loss (e.g. by forming the magnitude). Thus, if the acquired PT signal has the size (channels, samples), then the real valued matrix has the size (2* channels, samples). This may be done by concatenating the real and imaginary parts of the channel signals into the real valued matrix. In a next step, a real valued BSS algorithm, for example using PCA operations, is performed on the real valued matrix. The 1st N (2 to 5, preferably 2 to 3) strongest signal components (for example principal components or statistically independent components) are stored as weighting vectors. Thus, the result of applying these weighting vectors is a N-dimensional signal for each time point. This N-dimensional PT signal may also be called "vector cardiac Pilot Tone" signal or VCPT. The VCPT signal may be used to stabilise the signal by using more input data, i.e. signal components, and suppress and reject artefacts. Advantageously, the artefacts from RF pulses may be better detected and suppressed. Further, the detection of trigger points can be stabilised, because it may be based on multi-dimensional features, and not on a single feature of a one-dimensional signal. Because of these advantages, the VCPT will enable other trigger time points, especially an earlier one, thus improving robustness of dark blood and tagging measurements.

According to an embodiment, trigger time points for triggering the acquisition of the image data are determined by evaluating properties of the multi-dimensional Pilot Tone signal or of the time derivative of the multi-dimensional Pilot Tone signal, in particular by evaluating one or more of its position, direction, velocity, acceleration or change in direction in the multi-dimensional signal space.

The multi-dimensional Pilot Tone signal representing the cyclical cardiac movement may be represented in a multi-dimensional space having N dimensions. Since the cardiac movement is cyclical, the signal will move in the n-dimensional space in closed curves. For example, if the Pilot Tone signal is two-dimensional, it may for example be represented by real and an imaginary component, which may be plotted in the complex plane over multiple cardiac cycles. If there was only one, real valued component containing the Pilot Tone signal, the signal would have a linear shape, oriented in an arbitrary direction in the plane. However, in the complex plane, the signal displays a repetitive two-dimensional pattern, with characteristic features which may be used as trigger points. By retrospectively analysing such a multi-dimensional plot, one may derive suitable characteristic features from the n-dimensional signal pattern, which may serve as trigger time points for triggering the acquisition of the image data. These characteristic features are preferably detected practically in real time when acquiring further portions of the Pilot Tone signal. In real-time means that the trigger time point is only delayed by the necessary data transfer delays and signal processing delays, e.g., by group delays of applied low pass filters.

Instead of the n-dimensional Pilot Tone signal itself, one may also analyse the time derivative. It has been demonstrated that the time derivative displays a particularly distinguishable feature at the end of the diastole. Therefore, according to an advantageous embodiment, the time derivative of the multi-dimensional Pilot Tone signal is used for controlling, in particular triggering, the acquisition of the image data.

Thus, gating or trigger time points may be determined by evaluating properties of the n-dimensional Pilot Tone signal or of the time derivative of the n-dimensional Pilot Tone signal. Both of these signals may be plotted in n-dimensional space. The gating or trigger time points may be derived by evaluating one or more of the signal's position, direction, velocity, acceleration or change in direction in the multi-dimensional signal space. For example, the image acquisition may be triggered each time the n-dimensional Pilot Tone signal travels through a defined region in the n-dimensional space. Alternatively, it may be triggered by an acceleration phase, in particular when the acceleration follows a phase of slow motion through the signal space. This would for example indicate the beginning of contraction after the diastole. The trigger time points may also be determined by evaluating two different properties of the n-dimensional Pilot Tone signal, for example position and direction, position and change in direction, or position and acceleration. For example, trigger time point may be determined when the signal experiences a certain acceleration or change in direction when within a certain n-dimensional area of the n-dimensional signal space.

According to an embodiment, the acquisition of the image data is triggered at a defined time point within the cardiac cycle, in particular at a time point between 250ms before and 50ms after an R-wave, preferably between 200ms before the R-wave and the R-wave, most preferred between 150ms and 20ms before an R-wave. As explained above, with the inventive method, it is possible to detect a trigger time point even before the R-waves. It is hypothesised that the detected property of the n-dimensional PT signal is caused by the contraction of one atrium or both atria. It has been found that the inventive method is sensitive enough to detect a time point even before the R-wave and therefore a very useful trigger time point. Triggering before the R-wave is highly useful, since it allows to start the acquisition, for example magnetisation preparation pulses, even before the ventricle is contract.

According to an embodiment, the method comprises a further step of applying an adaptive or stochastic or model-based filter to the multi-dimensional Pilot Tone signal representing the cardiac movement, to obtain a filtered movement signal. The filter may advantageously determine in a stable way one or several of the signal's derivatives, in particular its first derivative (i.e., velocity). The Filter can be e.g. a Kalman filter, an extended Kalman filter, or a switched Kalman Filter. Possible filtering methods are disclosed in EP3413076 A1 and are incorporated herein by reference.

If the filtered movement signal is to be used in triggering, the filter may not introduce significant delay. Therefore, advanced filters like adaptive or stochastic or model-based filters are preferred. In preferred embodiments, the filter not only denoises the movement signal, but already assigns segments or specific points of the movement signal to the respective phases of the cardiac movement. Preferably, the adaptive or stochastic or model-based filter is first trained or adapted to the multi-dimensional PT signal derived from the training portion. Thus, the training portion of the Pilot Tone signal may be used also to configure the filter, since the filter may during this training phase generate or adapt a model of the movement signal to the actual Pilot Tone signal acquired in that particular measurement.

In other words, segmentation of the cardiac component enables triggering e.g. on the start/end of distinct cardiac phases. In addition, model-based segmentation is robust against measurement noise and may enable triggering on any, arbitrary points in the cardiac cycle. In the absence of severe arrhythmia, model-based filtering methods may also be able to predict cardiac activity beyond the current cardiac phase.

Preferably, the filter is a Kalman Filter, or an Extended Kalman filter, or is a Switched/Switching Kalman Filter, wherein the Switching Kalman Filter switches between several models during various phases of the cyclical movement. Kalman filtering, also known as linear quadratic estimation (LQE), is an algorithm that uses a series of measurements observed over time, containing statistical noise and other inaccuracies, and produces estimates of unknown variables that tend to be more accurate than those based on a single measurement alone, by using Bayesian inference and estimating a joint probability distribution over the variables for each timeframe. Thus, the Kalman filter provides, on the basis of the past measurements, e.g. the training portion, for each filtered data point a probably correct data point. The Switched Kalman filter may also include information on the physiological phase of the data point, i.e. may already perform segmentation. The Kalman, Extended Kalman and Switched/Switching Kalman filter make us of prior information trained on actual data. Thus, these and other model-based filters make use of a model of the movement signal. Once such a model has been generated, e.g. by analysis of the cardiac component trace acquired during the training phase, segmentation can be achieved by various methods, such as Hidden Markov Models or Switched Kalman Filters. These methods may also be used retrospectively to obtain segmentations of the cardiac component.

As described above, in a preferred embodiments the adaptive or stochastic or model-based filter automatically segments the N-dimensional PT signal into two or more sections corresponding to two or more physiological phases of the cyclical movement, in particular to the phases of the cardiac movement such as systole and diastole.

Accordingly, in preferred embodiments of the invention, the time points used for triggering a scan of medical data from the part of the human or animal body, or for post-processing a scan of medical data performed during the acquisition of the Pilot Tone signal, are extracted from the filtered n-dimensional PT signal. Preferably they are based on properties of the curve or on parameters of the stochastic or adaptive or model-based filter. In preferred embodiments, the time points used for triggering or post-processing can be directly derived from the stochastic or adaptive or model-based filter, once it has been trained.

According to an embodiment, the adaptive or stochastic or model-based filter is adapted to obtain properties of the multi-dimensional Pilot Tone signal, the properties of the multi-dimensional Pilot Tone signal being in particular a velocity vector or acceleration vector. As explained above, it has been found that the velocity and/or acceleration vector provides significant information which may be used to find the desired early trigger time point.

The invention is further directed to a computer program comprising program code, which induces a control unit of a radiological imaging modality to perform the method according to one of the preceding claims, when the program code is executed on the control unit. Preferably, the computer program can be started and ended by a user.

The invention is further directed to a digital storage medium comprising the program code as described. The digital storage medium can be in the form of a hard drive, like a magnetic hard disk or a solid state drive, or in the form of a portable storage medium like a CD, DVD or USB-Stick, or in the form of a network storage medium, like a NAS-storage or a cloud storage.

The invention is further directed to a control unit adapted for performing the method as described, wherein the control unit may be part of a radiological imaging modality, in particular a magnetic resonance system.

The invention is further directed to a radiological imaging modality, in particular a magnetic resonance system, comprising such a control unit. The magnetic resonance system is configured for performing the method of the invention.

Preferred embodiments of the invention shall now be described with reference to the appended drawings, in which:
- Fig. 1: shows a schematic flow diagram of an embodiment of the inventive method.
- Fig. 2: shows an example two-dimensional derivative of a cardiac PT signal in the complex plane.
- Fig. 3: shown an MR-scanner in a schematic view.

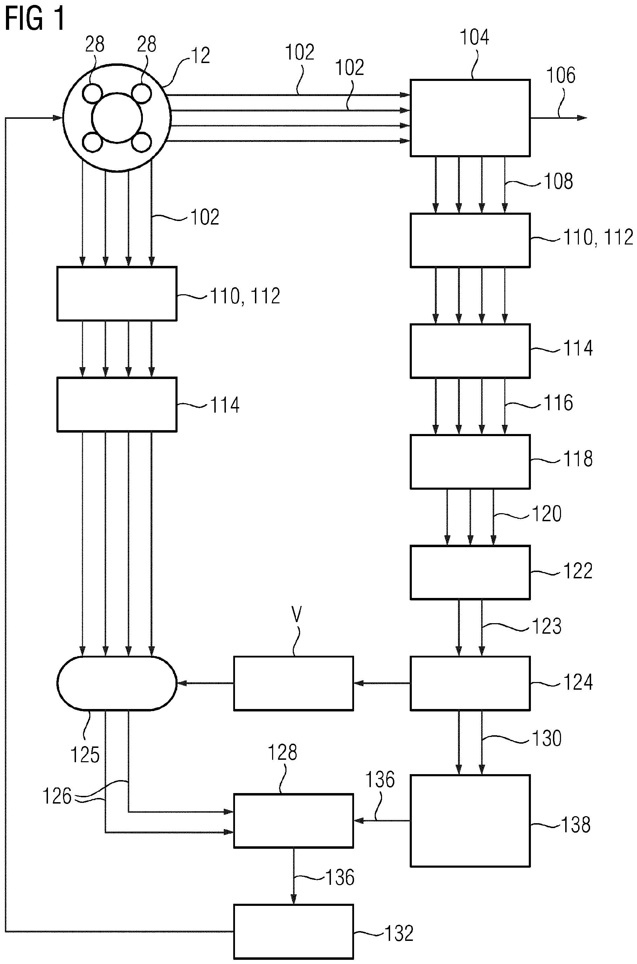

In the following and with reference to Fig. 1, we shall give an overview of an embodiment of the inventive method. In the example, we assume that a Pilot Tone signal is acquired during an MR scan. The MR scanner 12 including a receiver coil arrangement 28 with (in this schematic example) four coils/channels is shown on the top left. The MR scanner 12 also has Tx RF antenna for transmitting the PT signal. The receiver coil arrangement acquires a signal 102 having 4 channel signals, which is first subjected to a calibration step 104. The purpose of the calibration step is to separate the MR imaging signal from the additional Pilot Tone signal. The MR data 106 is further processed to produce MR image data, as is known in the art.

The Pilot Tone signal 108 comprising the 4 channel signals is optionally pre-processed by low-pass or bandpass filtering 110 (to avoid aliasing of high-frequency noise), followed by down-sampling 112. This is because the MR signal is acquired at a very high sampling rate, which is not required for the analysis of cardiac motion. The pre-processing is optional because it increases SNR (signal to noise ratio) but at the cost of additional time delay.

The pre-processed signal may further be subjected to a normalization step 114, in which the phases of all channels are normalised to a reference phase. The phase normalisation may be achieved by multiplying with the complex conjugate of the reference channel, i.e. one of the channels is selected as the reference channel.

The normalised, complex Pilot Tone channel signals 116 are then further processed to separate the various motion components modulating the Pilot Tone signal. This is done first by Principle Component Analysis 118, in which the largest principle components 120 corresponding to respiratory movement are extracted, as described above. They are then removed from the channel signals, and the respiration-free channel signals are subjected to a further BSS algorithm, e.g. an Independent Component Analysis 122. Through the ICA, the strongest N independent signal components 123 corresponding to the cardiac movement are identified and separated. Preferably, the number N is predefined. The corresponding weighting vectors V for each independent signal component corresponding to the cardiac movement are calculated in step 124. The step 124 is preferably done automatically, e.g. by calculating the signal energy in the cardiac motion band for each independent component, compared to the signal energy in other frequency bands, and selecting the components with the highest relative signal energy in the cardiac motion band. Alternatively, the degree of correlation of each signal component with a typical cardiac component trace may be calculated. Once the strongest N independent components representing the cardiac motion have been selected, the weighting vectors V can be automatically calculated. The weighting vectors V correspond to a linear combination of the channel signals 102/108 of the several receiver channels. The weighting vectors V are applied to the training portion of the PT signal to obtain the VCPT signal portion 130. The time derivative of the VCPT signal is then determined, preferably by a constant velocity Kalman filter. The further processing, in particular the detection of trigger points, is carried out on the derivative. Thus, the time derivative of the VCPT signal portion 130 is analysed in step 138 to determine suitable trigger time points 136. For example, the time derivative of the VCPT signal 130 may be averaged over several heart cycles and searched for characteristic features, such as an acceleration or change in direction in the n-dimensional signal space. This feature is then used in step 132 to detect the trigger time point on the incoming further Pilot tone signal data 102.

The weighting vectors V are stored and applied to the incoming further Pilot Tone signal data 102. In some embodiments, the incoming data 102 may first be subjected to at least one of low-pass filtering, extrapolation across invalid samples, e.g., during RF pulses, and down-sampling 110/112, as well as phase-normalization 114. The normalised complex channel signals are then multiplied with the weighting vectors V in step 125 to obtain the N-dimensional Pilot Tone signal 126 corresponding to the cardiac movement.

The N-dimensional Pilot Tone signal 126 may be subjected to a model-based filter 128 as described above. In some embodiments, the filter 128 is first trained on a training portion of the PT signal. The above-described adaptive filters like the Kalman Filters and Switched Kalman Filters need some time to converge, thus a calibration is useful to ensure fast convergence, but not absolutely necessary. In other applications, the filter 128 adapts over time to the incoming movement signal 126 and does not require a calibration.

In the step 128, the trigger time points are detected and used to trigger the MR acquisition in the MR scanner 12.

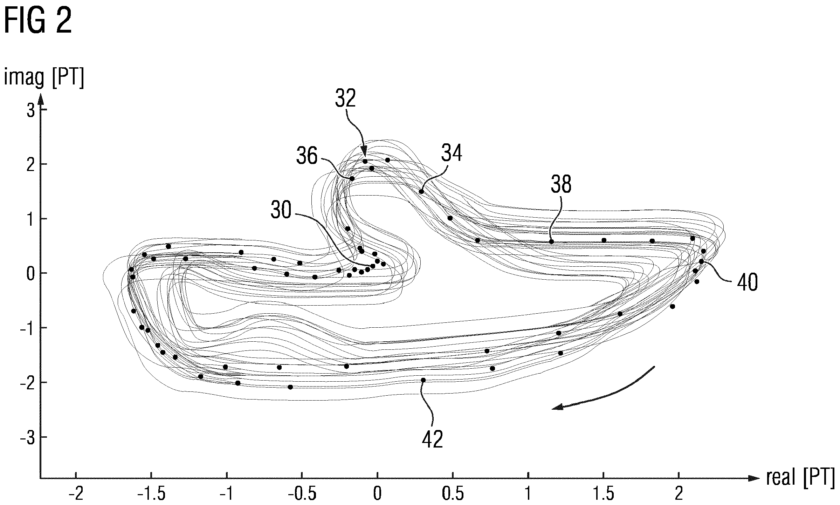

Fig. 2 depicts a two-dimensional PT signal plotted over multiple heart cycles in the complex plane. In particular, it shows the real and imaginary part of the time derivative of the first (strongest) cardiac component. Samples at a time resolution of 25ms are plotted as small circles, and the direction of the signal is indicated by the arrow. The signal displays a repetitive two-dimensional pattern with a well-defined change of direction 30 at about the end of the diastole (0,0). This is followed by a well-defined "notch" 32. The time point of the R-wave is indicated at 34. Taking into account the time delay of about 100ms caused by the low pass filter delay, it is possible to set a trigger point at or before the R-wave, e.g. by triggering at the distinctive change of direction at point 30, or somewhat afterwards during the also distinctive notch 32. For example, setting the trigger point 36 will result in a trigger precisely at the R-wave. By contrast, the currently used trigger point is at 38, thus well after the R-wave at about 40% maximum contraction speed around (1,0). Therefore, by including the second cardiac component, earlier trigger time points 30 or 36 can be realized. Point 40 indicates the mid-systolic point of maximum contraction speed, and point 42 the end-systole, where the contraction is maximal.

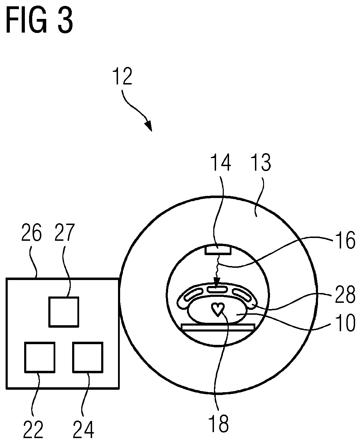

Fig. 3 finally shows a schematic MR scanner 12. The MR-scanner 12 includes a main magnet 13 and a control unit 24, by which the data acquisition of the MR scanner 12 can be controlled. The control unit 24 can be part of a computer device 26. The computer device can also include a digital storage medium 22 and a user interface 27 including e.g. a display, a keyboard, mouse, touch screen or such like. A patient 10 may be examined, in particular in order to perform MR imaging of the heart 18.

A pilot tone signal 16 is emitted by a pilot tone emitter 14, which may be a separate RF source. Preferably, the pilot tone emitter 14 is positioned close to the heart, e.g. strapped to the local coil 28 or included in the coil. The pilot tone signal is modulated by the movement of the heart 18 and the lung (not shown). The (modulated) pilot tone signal is received by the receiver coil arrangement 28, which is preferably a local coil 28, such as an anterior (spine) coil, a head coil or chest array coil, but may also be the body coil, possibly including anterior (spine) coils.

## Claims

1. A method for acquiring image data in a radiological examination of a part of the human or animal body, wherein the part is subjected to a cardiac movement, the method comprising the steps:
a) transmitting a radiofrequency Tx Pilot Tone signal via at least one RF transmit antenna,
b) Receiving a Pilot Tone signal (108) from the body part via a radiofrequency receiver coil arrangement (28) comprising several channels, wherein the received Pilot Tone signal comprises several channel signals (102) associated with the several channels;
c) Carrying out a blind source separation algorithm (122) on a training portion of the Pilot Tone signal and thereby determining weighting vectors (V) to extract cardiac movement signals in the presence of other signals, wherein the weighting vectors (V) allow to form weighted combinations of the several channel signals (102);
d) Selecting and storing at least two non-parallel weighting vectors (V) which allow to extract signal components (123), which represent cardiac movement, from the several channel signals;
e) Applying the weighting vectors to the further portions of the Pilot Tone signal (102) to obtain a multi-dimensional Pilot Tone signal (126) representing the cardiac movement, wherein the multi-dimensional Pilot Tone signal has at least two dimensions;
f) Using the multi-dimensional Pilot Tone signal (126) for controlling, in particular triggering, the acquisition of the image data, or for retrospectively gating or correcting the acquired image data.

2. The method of claim 1, wherein the multi-dimensional Pilot Tone signal (126) representing the cardiac movement has between 2 and 5, preferably 2 or 3, dimensions.

3. The method of any one of the preceding claims, wherein the blind source separation algorithm utilises one or more Principal Component Analysis operations (118).

4. The method of any one of the preceding claims, wherein the blind source separation algorithm utilises one or more Independent Component Analysis (122) operations.

5. The method of any one of the preceding claims, wherein the blind source separation algorithm is used to detect the strongest independent component corresponding to the cardiac movement, and the method comprises
• using the strongest independent component to retrospectively analyse the training portion of the Pilot Tone signal, in particular to average the Pilot Tone signal over a plurality of cardiac intervals, the cardiac intervals having been determined from the strongest independent component, and
• detecting at least one further independent component corresponding to the cardiac movement from the retrospective analysis.

6. The method of any one of the preceding claims, wherein the channel signals (102) of the received Pilot Tone signal are complex-valued, and wherein the weighting vectors each extract a real or an imaginary part of a signal component.

7. The method of any one of the preceding claims, wherein the channel signals (102) of the received Pilot Tone signal are complex-valued, and wherein the channel signals are rotated in the complex plane before carrying out the blind source separation algorithm, in particular rotated so that a mean of the rotated signal lies on one of the diagonals of the complex plane.

8. The method of any one of the preceding claims, wherein the channel signals (102) of the received Pilot Tone signal are complex and the blind source separation algorithm is real-valued, and wherein the method comprises
- generating a real-valued matrix in which the real and imaginary parts of the complex channel signals form separate channels,
- performing a blind source separation algorithm on the real-valued matrix.

9. The method of any one of the preceding claims, wherein the time derivative of the multi-dimensional Pilot Tone signal (126) is used for controlling, in particular triggering, the acquisition of the image data.

10. The method of any one of the preceding claims, wherein trigger time points (30, 36) for triggering the acquisition of the image data are determined by evaluating properties of the multi-dimensional Pilot Tone signal or of the time derivative of the multi-dimensional Pilot Tone signal, in particular by evaluating one or more of its position, direction, velocity, acceleration or change in direction in the multi-dimensional signal space.

11. The method of any one the preceding claims, wherein the acquisition of the image data is triggered at a defined time point (30, 36) within the cardiac cycle, in particular at a time point between 250ms before and 50ms after an R-wave, preferably between 200ms before the R-wave and the R-wave, most preferred between 150ms and 20ms before an R-wave.

12. The method of any one the preceding claims, comprising a further step of applying an adaptive or stochastic or model-based filter (128) to the multi-dimensional Pilot Tone signal representing the cardiac movement, to obtain a filtered movement signal (30, 130).

13. The method of claim 12, wherein the adaptive or stochastic or model-based filter is adapted to obtain properties of the multi-dimensional Pilot Tone signal, the properties of the multi-dimensional Pilot Tone signal being in particular a velocity vector or acceleration vector.

14. A computer program comprising program code, which induces a control unit (24) of a radiological imaging modality (12) to perform the method according to one of the preceding claims, when the program code is executed on the control unit.

15. A control unit (24) adapted for performing the method according to one of claims 1 to 13, wherein the control unit may be part of a radiological imaging modality, in particular a magnetic resonance system (12).
